(19) Europäisches Patentamt

European Patent Office

Office européen des brevets

(11) **EP 0 881 920 B1**

(12) **FASCICULE DE BREVET EUROPEEN**

(45) Date de publication et mention
de la délivrance du brevet:
**25.09.2002 Bulletin 2002/39**

(21) Numéro de dépôt: **97928334.8**

(22) Date de dépôt: **11.06.1997**

(51) Int Cl.⁷: **A61L 27/00**, A61K 6/033,
A61L 31/00

(86) Numéro de dépôt international:
**PCT/FR97/01045**

(87) Numéro de publication internationale:
**WO 97/047334 (18.12.1997 Gazette 1997/54)**

(54) **PROCEDE DE PREPARATION D'UN MATERIAU COMPOSITE IMPLANTABLE, MATERIAU OBTENU, IMPLANT COMPRENANT CE MATERIAU ET KIT DE MISE EN OEUVRE**

VERFAHREN ZUR HERSTELLUNG EINES IMPLANTIERBAREN VERBUNDWERKSTOFFS, SO HERGESTELLTE WERKSTOFFE, UND KIT ZUR DURCHFÜHRUNG DES VERFAHRENS

METHOD FOR PREPARING AN IMPLANTABLE COMPOSITE MATERIAL, RESULTING MATERIAL, IMPLANT INCLUDING SAID MATERIAL, AND KIT THEREFOR

(84) Etats contractants désignés:
**AT BE CH DE DK ES FI FR GB GR IE IT LI LU MC NL PT SE**

(30) Priorité: **14.06.1996 FR 9607590**

(43) Date de publication de la demande:
**09.12.1998 Bulletin 1998/50**

(73) Titulaire: **Depuy Bioland
31100 Toulouse (FR)**

(72) Inventeurs:
• **LERCH, Alain
F-31400 Toulouse (FR)**
• **FRAYSSINET, Patrick, Lieut-dit Le Gaillard
F-31470 Saint-Lys (FR)**

(74) Mandataire:
**Cabinet BARRE LAFORGUE & associés
95, rue des Amidonniers
31000 Toulouse (FR)**

(56) Documents cités:
EP-A- 0 360 244      EP-A- 0 639 366
DE-A- 4 302 072      US-A- 4 626 392
US-A- 5 091 344

• PATENT ABSTRACTS OF JAPAN vol. 016, no.
029 (C-0904), 24 janvier 1992 & JP 03 242364 A
(ASAHI OPTICAL CO LTD), 29 octobre 1991,

**Description**

L'invention concerne un procédé de préparation d'un matériau composite solide destiné à être implanté dans un milieu biologique humain ou animal, notamment à titre de substitut osseux ou dentaire ; un matériau ainsi obtenu ; un implant comprenant un tel matériau et un kit de mise en oeuvre extemporanée de ce procédé.

Dans tout le texte de la présente demande, le terme "implant" désigne tout dispositif, appareil, mécanisme, pièce ou ensemble de pièces, d'origine naturelle ou artificielle, organique ou inorganique, à l'état solide, biocompatible et implantable dans le corps humain ou animal, à l'exclusion des compositions fluides, pâteuses ou divisées.

A titre d'exemples d'implants, on peut citer les prothèses osseuses, dentaires ou maxillofaciales, les inserts de comblement, d'obturation ou d'interface,...

L'utilisation de céramiques poreuses à pores interconnectés à titre de matériau solide implantable pour substitut osseux a déjà été décrite (voir par exemple EP-A-0 360 244 et "Osseointegration of macroporous calcium phosphate ceramics having a different chemical composition" Frayssinet et al., Biomaterials 1993, Vol. 14, n°6, p 423 - 429). En raison même de leur macroporosité, les matrices solides poreuses biocompatibles implantables telles que les céramiques, et plus particulièrement les matrices à pores interconnectés, présentent l'avantage d'augmenter La surface d'échange avec le milieu biologique, d'être biorésorbables, de faciliter la revascularisation des tissus et de posséder d'excellentes propriétés ostéoconductrices. En outre, on peut déposer par précipitation des agents de croissance dans les pores.

Néanmoins, ces céramiques poreuses sont par nature fragiles et friables et ne peuvent pas aisément être mises en forme par le praticien lors de l'opération. En outre, il n'est pas non plus possible de fixer des vis ou chevilles dans ces céramiques. De ce fait, l'utilisation pratique de ces céramiques est aujourd'hui limitée aux rares cas où il est certain, à l'avance, que le matériau n'aura pas à être retaillé et ajusté extemporanément, ni à supporter des vis de fixation.

En outre, les céramiques poreuses présentent une faible résistance mécanique (2 à 4 MPa en compression), insuffisante dans de nombreuses applications, par exemple lorsqu'une remise en charge du site d'implantation avant cicatrisation est nécessaire.

Par ailleurs, on connaît (par exemple EP-A-0 639 366) des ciments hydrauliques phosphocalciques biocompatibles implantables, c'est-à-dire des compositions fluides ou pâteuses durcissables, permettant de réaliser des montages prothétiques ou des comblements lors d'opérations chirurgicales orthopédiques. Ces ciments sont facilement malléables par le praticien, mais ne peuvent pas être utilisés pour réaliser des implants solides tels que des prothèses osseuses dont la forme générale doit être prédéfinie, qui doivent résister dès l'implantation à de fortes contraintes et/ou dans lesquels on doit visser des vis pour la fixation d'autres éléments ou organes. Ainsi, ces ciments ne sont utilisés que pour coller des implants ou organes entre eux, ou parfois comme matériau de comblement dans les sites non exposés à de fortes contraintes.

Dans ce contexte, l'invention vise à proposer un matériau composite et son procédé de préparation, qui est destiné à être implanté dans un milieu biologique (corps humain ou animal), qui présente les avantages des céramiques poreuses à pores interconnectés (résorption rapide et complète, ostéoconduction...), mais qui présente des propriétés mécaniques améliorées.

L'invention vise en particulier à proposer un matériau composite implantable apte à être utilisé dans la composition d'un implant, et qui peut être facilement mis en forme, taillé, coupé, fraisé..., notamment par le praticien lors de la pose de l'implant.

L'invention vise aussi à proposer un tel matériau apte à subir des contraintes dès l'implantation.

L'invention vise aussi à proposer un tel matériau dans lequel on peut insérer et fixer des chevilles ou des vis telles que des vis auto-foreuses et qui procure un bon maintien de ces vis.

L'invention vise aussi à proposer un matériau composite implantable résorbable par un milieu biologique, et dont la résistance en compression est équivalente à celle de l'os naturel, notamment supérieure à 10 MPa, plus particulièrement de l'ordre de 20 MPa.

L'invention vise aussi à proposer un tel matériau composite dont la cinétique de résorption dans le milieu biologique peut être connue et ajustée avant l'implantation.

L'invention vise aussi à proposer un procédé de préparation d'un tel matériau composite.

L'invention vise également à proposer un implant prothétique osseux comprenant un matériau présentant les avantages sus-mentionnés.

L'invention vise également à proposer un procédé de fabrication de cet implant.

L'invention vise plus particulièrement à proposer un procédé de préparation d'un matériau composite et un procédé de fabrication d'implants, notamment de substituts osseux ou dentaires, particulièrement simples, rapides et qui peuvent être mis en oeuvre au moins partiellement par le praticien peu de temps avant l'opération, voire même pendant l'opération.

Pour ce faire, l'invention concerne un procédé de préparation d'un matériau composite destiné à être implanté dans un milieu biologique humain ou animal, caractérisé en ce que :

.    on choisit ou on prépare préalablement au moins un bloc d'une matrice solide poreuse biocompatible comprenant majoritairement au moins un sel de calcium choisi parmi les phosphates de calcium, les sulfates de calcium et les carbonates de calcium,

. on prépare, indépendamment de la matrice solide, une solution liquide durcissable adaptée pour former, après durcissement, une composition solide biocompatible durcie d'un ciment hydraulique comprenant majoritairement au moins un sel de calcium choisi parmi les phosphates de calcium, les sulfates de calcium et les carbonates de calcium, et qui est adaptée pour être entièrement résorbable par le milieu dans lequel l'implant doit être implanté,

. on plonge le bloc de matrice dans la solution liquide, avant son durcissement, pendant une durée adaptée pour que celle-ci imprègne et remplisse le volume poreux du bloc de matrice à coeur,

. on fait ensuite sécher et durcir la solution liquide en composition solide.

Avantageusement et selon l'invention, on met ensuite en forme chaque bloc de matériau composite ainsi obtenu.

Avantageusement et selon l'invention, on choisit ou on prépare au moins un bloc entièrement poreux notamment macroporeux- et de préférence de porosité au moins sensiblement homogène dans tout le volume du bloc.

L'invention concerne aussi un matériau composite obtenu par un procédé selon l'invention. Ainsi, l'invention concerne un matériau composite destiné à être implanté dans un milieu biologique humain ou animal caractérisé en ce qu'il comprend :

a) une matrice solide poreuse biocompatible ayant une composition chimique comprenant majoritairement au moins un sel de calcium choisi parmi les phosphates de calcium, les sulfates de calcium et les carbonates de calcium,

b) une composition solide, compacte, non divisée, biocompatible d'un ciment hydraulique durci comprenant majoritairement au moins un sel de calcium choisi parmi les phosphates de calcium, les sulfates de calcium et les carbonates de calcium, cette composition solide remplissant le volume poreux de la matrice et étant adaptée pour être entièrement résorbable dans ledit milieu.

Avantageusement et selon l'invention, la composition solide est choisie pour présenter elle-même une résistance à la compression supérieure à 10 MPa, notamment comprise entre 20 MPa et 50 MPa.

Par ailleurs, avantageusement et selon l'invention, la composition solide est choisie pour présenter un module d'Young compris entre 700 MPa et 1000 MPa.

Plus particulièrement et selon l'invention, la composition solide comprend majoritairement du phosphate dicalcique dihydraté $CaHPO_4, 2H_2O$ (DCPD).

En outre, avantageusement et selon l'invention, la composition solide est du type obtenu par mélange d'une poudre de phosphate tricalcique β dans une solution d'acide orthophosphorique que l'on laisse durcir à température ambiante.

Avantageusement et selon l'invention, la matrice est également adaptée pour être entièrement résorbable dans ledit milieu, et la composition solide est adaptée pour présenter une cinétique de résorption dans ledit milieu plus rapide que celle de la matrice ; et la composition solide est adaptée pour être résorbable dans ledit milieu en une durée inférieure à trois mois, notamment de l'ordre de quelques semaines.

Avantageusement et selon l'invention, la matrice est une matrice minérale entièrement poreuse, notamment une céramique macroporeuse (c'est-à-dire dont la porosité est supérieure à sa porosité théorique et dont les pores ont une dimension moyenne comprise entre 50 μm et 2000 μm), de préférence à pores interconnectés. La dimension moyenne des pores est avantageusement comprise entre 200 μm et 500 μm.

Avantageusement et selon l'invention, la matrice présente une porosité au moins sensiblement homogène dans tout son volume, qui peut être comprise entre 5 % et 85 %, notamment entre 50 % et 85 %.

Avantageusement et selon l'invention, la matrice est choisie pour présenter une résistance à la compression comprise entre 1 MPa et 6 MPa, et un module d'Young compris entre 10 MPa et 200 MPa.

Ainsi, avantageusement et selon l'invention, la matrice comprend une proportion X en poids d'hydroxyapatite (HAP) $Ca_{10} (PO_4)_6 (OH)_2$, et une proportion Y en poids de phosphate tricalcique $Ca_3 (PO_4)_2$. Avantageusement et selon l'invention, le matériau est caractérisé en ce que X + Y = 100 %. Par exemple et selon l'invention, X est de 75 % et Y de 25 %.

Avantageusement et selon l'invention, la matrice et/ou la composition solide (ciment) incorpore(nt) au moins un agent bioactif. Grâce à l'invention, la cinétique de libération in situ d'un tel agent bioactif peut être contrôlée aisément puisqu'elle correspond au moins sensiblement à la cinétique de résorption de la matrice et/ou de la composition solide. Selon l'invention, on peut donc incorporer dans la matrice un agent bioactif dont la cinétique de libération doit être lente, par exemple prolongée sur plusieurs mois, et dans le ciment un agent bioactif dont la cinétique de libération doit être rapide, par exemple limitée à quelques semaines.

L'invention concerne également un implant, et plus particulièrement un substitut osseux ou dentaire solide, qui est caractérisé en ce qu'il comprend un matériau selon l'invention.

Avantageusement, un implant selon l'invention est caractérisé en ce qu'il comprend au moins une portion constituée d'un matériau selon l'invention destinée à être placée au contact d'un milieu biologique humain ou animal, notamment d'un tissu osseux.

L'invention concerne aussi un kit de mise en oeuvre extemporanée d'un procédé selon l'invention, caractérisé en ce qu'il comprend au moins un bloc d'une matrice solide poreux biocompatible et une dose de chacun des constituants nécessaires pour obtenir ladite solution liquide formant, après durcissement, la composition solide biocompatible. Le kit selon l'invention comprend par exemple un bloc de céramique macroporeuse phosphocalcique, une dose de poudre de phosphate tricalcique β avec 2,5 % de pyrophosphate de sodium anhydre et une dose de solution d'acide orthophosphorique de concentration comprise entre 1 et 5 mol/l, notamment entre 3,5 et 4,5 mol/l. Le rapport de la masse (en g) de poudre sur le volume (en ml) de la dose d'acide est compris entre 0,5 et 5, notamment entre 1 et 2.

L'invention concerne aussi un matériau, un implant, un procédé, et un kit, comprenant en combinaison tout ou partie des caractéristiques mentionnées ci-dessus ou ci-après.

La description qui suit présente des exemples de préparation d'un matériau et d'un implant conformes à l'invention.

EXEMPLE 1 :

1) On prépare dans un mélangeur de 15 l, une barbotine comprenant 1625 g d'hydroxyapatite en poudre et 875 g de phosphate tricalcique β en poudre, 45 g de polyélectrolite synthétique sans alcalis (dispersant liquide), 30 g de polyoxyde d'éthylène en poudre (liant organique), et 1165 ml d'eau.

On plonge des cylindres de 10 mm de diamètre et 10 mm de hauteur d'une mousse de polyuréthanne dans cette barbotine et on imprègne chaque cylindre de mousse à coeur par malaxage jusqu'à obtenir un taux d'imprégnation (rapport de la masse de barbotine sur la masse de mousse) de 25.

On place la mousse imprégnée dans un four à une température de 1150 °C pendant 15 min. Après refroidissement du four, on obtient une céramique macroporeuse, les composants organiques s'étant évaporés. Cette céramique présente une porosité de 75 %. Il est connu que cette céramique est entièrement résorbable en milieu biologique au bout d'environ 18 mois.

La résistance à la compression de la céramique est mesurée en soumettant un cylindre de 10 mm de diamètre et 10 mm de hauteur à un essai de compression. La résistance à la compression est de 1 MPa et le module d'Young est de 20 MPa.

2) On prépare une poudre de phosphate tricalcique β (TCP) par réaction à 1150 °C pendant 180 min d'hydroxyapatite et de phosphate dicalcique dihydraté.

On mélange 2,954 g de TCP avec 0,046 g de pyrophosphate de sodium anhydre en poudre.

On verse ces 3 g de poudre dans 2,3 ml de solution aqueuse d'acide orthophosphorique 4 M et d'acide sulfurique 0,1 M.

Après 30 s à 1 min de réaction, on obtient un mélange homogène de solution liquide durcissable.

3) On immerge 4 cylindres de la céramique macroporeuse préparée en 1) dans cette solution liquide durcissable pendant une durée de l'ordre de 20 s, jusqu'à ce que toutes les bulles d'air soient évacuées des pores.

4) On retire les cylindres imprégnés de la solution et on les laisse sécher à l'air libre. On constate un durcissement de la solution au bout d'environ 5 min en une composition solide, compacte, non divisée remplissant le volume poreux de la céramique.

Après cinq jours (évaporation de l'eau), on réalise des essais de compression. La résistance en compression obtenue est de 15 MPa et le module d'Young est de 400 MPa.

5) On constate que les cylindres peuvent être coupés à la scie en laissant un état de surface parfait.

On réalise un avant-trou par perçage dans un cylindre et on introduit une vis d'ostéosynthèse dans cet avant-trou à l'aide d'un tourne-vis. On constate que le matériau conserve sa cohésion et n'est pas détérioré, notamment autour de la vis qui est parfaitement maintenue en place.

6) On utilise la solution durcissable obtenue en 2) pour préparer des cylindres de 5 mm de diamètre et 10 mm de hauteur que l'on implante dans les condyles d'une série de 12 lapins.

Les lapins ont été sacrifiés à 15 jours, 6 semaines, 10 semaines et 16 semaines après implantation et les sites d'implantation ont été examinés histologiquement.

Il a été constaté que dans les deux premières semaines, une apposition de trabécules osseux se fait à la surface de l'implant. Des grains de matériaux sont retrouvés dans le tissu stomal présent entre les trabécules ainsi que dans les trabécules néoformés.

A six et neuf semaines, les cylindres sont fragmentés et des trabécules osseux ont pénétré dans tout le volume des cylindres entourant les fragments. Ces fragments sont en contact du tissu osseux néoformé sans aucune interposition fibreuse. De très nombreux fragments sont en voie d'être totalement intégrés dans le tissu osseux.

A treize semaines, le phénomène de fragmentation est accentué ainsi que celui d'intégration osseuse. Plus de la moitié du volume de l'implant a disparu à cette date.

En conclusion, la composition solide imprégnant des pores est entièrement résorbable dans un milieu biologique et présente une cinétique de résorption plus rapide que la matrice.

EXEMPLE 2 :

1) On prépare dans un mélangeur de 15 l, une barbotine comprenant 1625 g d'hydroxyapatite en poudre et 875 g de phosphate tricalcique β en poudre, 45 g de polyélectrique synthétique sans alcalis (dispersant liquide), 30 g de polyoxyde d'éthylène en poudre (liant organique), et 1165 ml d'eau.

On plonge des cylindres de 10 mm de diamètre et 10 mm de hauteur d'une mousse de polyuréthanne dans cette barbotine et on imprègne chaque cylindre de mousse à coeur par malaxage jusqu'à obtenir un taux d'imprégnation (rapport de la masse de barbotine sur la masse de mousse) de 30.

On place la mousse imprégnée dans un four à une température de 1150 °C pendant 15 min. Après refroidissement du four, on obtient une céramique macroporeuse, les composants organiques s'étant évaporés. Cette céramique présente une porosité de 68 %.

La résistance à la compression de la céramique est mesurée en soumettant un cylindre de 10 mm de diamètre et 10 mm de hauteur à un essai de compression. La résistance à la compression est de 3 MPa et le module d'Young est de 80 MPa.

On prépare ensuite, suivant les mêmes étapes 2) 3), et 4) que dans l'exemple 1, un matériau composite selon l'invention. Les mêmes essais et constatations que celles mentionnées en 4) et 5) dans l'exemple 1 sont faits, avec les mêmes résultats, avec le matériau composite obtenu dans cet exemple 2. Après cinq jours (évaporation de l'eau), on réalise des essais de compression. La résistance en compression obtenue est de 20 MPa et le module d'Young est de 500 MPa.

EXEMPLE 3 :

On prépare un matériau composite selon l'invention comme à l'exemple 2, à partir d'une matrice macroporeuse de phosphate de calcium constituée de 75 % (en poids) d'hydroxhyapatite, et 25 % (en poids) de phosphate tricalcique β. La porosité de cette matrice est de 70 % et la taille moyenne des pores mesurée est de 500 μm. Tous les pores sont interconnectés. Cette matrice est immergée dans une solution liquide durcissable de DCPD.

Le matériau composite obtenu après durcissement est formé de cylindres de 8 mm de diamètre et 15 mm de longueur que l'on implante dans des trous de 9 mm réalisés dans les condyles externes de 12 moutons. Sur chaque animal, on implante dans un condyle fémoral un cylindre de matériau composite selon l'invention, et sur l'autre condyle, un cylindre de même dimension formé uniquement de la matrice de céramique macroporeuse de phosphate de calcium utilisée pour réaliser le matériau composite selon l'invention, c'est-à-dire, telle qu'elle se présente avant immersion dans la solution du liquide durcissable.

Quatre moutons sont sacrifiés successivement à 20 jours, 60 jours, 120 jours après l'implantation, et les sites d'implantation sont examinés histologiquement.

Au bout de 20 jours, aucun des implants n'a été ostéointégré. Quelques trabécules de tissu osseux se forment dans le tissu entourant les implants. Ces trabécules proviennent généralement des boras de la cavité osseuse et s'étendent vers l'implant. Les pores des implants formés de matrice seule sont envahis par du tissu fibreux, et des trabécules sont parfois trouvés en contact avec la surface externe de la matrice céramique.

Concernant les cylindres de matériau composite selon l'invention, des trabécules osseux sont souvent formés à la surface de l'implant et certains sont insérés dans le ciment qui montre des marques de dégradation dans ces zones. Quelques grains ayant une dimension de quelques micromètres ont été éliminés, ce qui forme des vides dans les pores cimentés dans lesquels une matrice extra-cellulaire s'est formée. Ces grains ont été phagocytés par des macrophages mononucléés à la périphérie de l'implant.

A 60 jours, les implants de matrice seule sont partiellement intégrés, avec une croissance osseuse à la surface des pores externes. La partie centrale de la matrice contient toujours du tissu fibreux dans lequel des cellules mononucléées contenant des particules de céramiques sont visibles.

Concernant les implants de matériau composition selon l'invention, on constate une croissance progressive de tissu osseux dans les pores externes au sein desquels le ciment a été lentement remplacé par le tissu osseux. La plupart des cristallites de ciment situées à proximité de la surface sont revêtues d'une substance protéique. Ces cristallites ont été phagocytées et dégradées par des macrophages. De nombreux ostéoblastes se distinguent à la surface des agrégats minéraux. La localisation des ostéoblastes à la surface semble démontrer un processus de différenciation préférentielle. Tous les fragments de ciment n'ont pas été dégradés par les macrophages. Certains sont incorporés dans la matrice osseuse qui a été formée dans les pores. Une certaine dégradation de la matrice céramique est également constatée.

A 120 jours, une croissance du tissu osseux dans la totalité des cylindres a été constatée avec une dégradation majeure. La totalité des pores des implants de matériau selon l'invention ne comprennent plus de ciment, celui-ci se retrouvant dans les particules minérales dans des macrophages. Quelques îlots de macrophages sont présents dans la cavité de moelle osseuse du tissu osseux. Des îlots de macrophage ont phagocyté le tissu osseux, mais aucune résorption exagérée d'os n'a pu être constatée à proximité immédiate des implants.

Par contre, les implants de matrice céramique seule sont totalement intégrés et présentent des signes de résorption.

Les mesures histomorphométriques indiquent que la quantité d'os et le taux d'ossification sont plus importants dans les implants de matériau composite selon l'invention que dans les implants formés de matrice céramique poreuse seule.

Ces essais démontrent que la formation d'un tissu osseux à la surface du matériau selon l'invention est précédée d'un dépôt de matrice de protéines qui pourrait n'être pas toujours associé à une synthèse par des cellules ostéogéniques à proximité immédiate. Cette matrice qui a pénétré les micropores du matériau n'était pas minéralisée et semblait être un préalable à la formation osseuse par les ostéoblastes.

Le processus de formation osseuse est très actif dans les zones de dégradation du ciment. La présence des macrophages qui avaient phagocyté les cristaux de phosphate de calcium n'a pas déclenché l'activation des ostéoclastes comme le démontre l'absence d'ostéolyse du tissu osseux qui a envahi la céramique. De plus, il semble que la formation d'os a été accrue par la présence du ciment sans que l'on puisse donner une explication claire à ce phénomène. Une explication possible serait que ce phénomène soit lié à la dégradation du ciment de phosphate de calcium. La complexité de l'activation des macrophages et le grand nombre de produits qui peuvent être synthétisés par ces cellules pourrait expliquer également partiellement ces résultats.

En conclusion, le matériau composite selon l'invention présente aussi bien des propriétés mécaniques que des propriétés biologiques améliorées.

## Revendications

1. Procédé de préparation d'un matériau composite destiné à être implanté dans un milieu biologique humain ou animal, **caractérisé en ce que** :

   - on choisit ou on prépare préalablement au moins un bloc d'une matrice solide poreuse biocompatible comprenant majoritairement au moins un sel de calcium choisi parmi les phosphates de calcium, les sulfates de calcium et les carbonates de calcium,
   - on prépare, indépendamment de la matrice solide, une solution liquide durcissable adaptée pour former, après durcissement, une composition solide biocompatible durcie d'un ciment hydraulique comprenant majoritairement au noms un sel de calcium choisi parmi les phosphates de calcium, les sulfates de calcium et les carbonates de calcium, et qui est adaptée pour être entièrement résorbable par le milieu dans lequel l'implant doit être implanté,
   - on plonge le bloc de matrice dans la solution liquide, avant son durcissement, pendant une durée adaptée pour que celle-ci imprègne et remplisse le volume poreux du bloc de matrice à coeur,
   - on fait ensuite sécher et durcir la solution liquide en composition solide.

2. Procédé selon la revendication 1, **caractérisé en ce qu'**on met ensuite en forme chaque bloc de matériau composite ainsi obtenu.

3. Matériau composite destiné à être implanté dans un milieu biologique humain ou animal, susceptible d'être obtenu par un procédé selon l'une des revendications 1 et 2, **caractérisé en ce qu'**il comprend :

   a) une matrice solide poreuse biocompatible ayant une composition chimique comprenant majoritairement au moins un sel de calcium choisi parmi les phosphates de calcium, les sulfates de calcium et les carbonates de calcium,
   b) une composition solide, compacte, non divisée, biocompatible d'un ciment hydraulique durci comprenant majoritairement au moins un sel de calcium choisi parmi les phosphates de calcium, les sulfates de calcium et les carbonates de calcium, cette composition solide remplissant le volume poreux de la matrice et étant adaptée pour être entièrement résorbable dans ledit milieu.

4. Matériau selon la revendication 3. **caractérisé en ce que** la composition solide est choisie pour présenter une résistance à la compression supérieure à 10 MPa, notamment comprise entre 20 MPa et 50 MPa.

5. Matériau selon l'une des revendications 3 et 4, **caractérisé en ce que** la composition solide est choisie pour présenter un module d'Young compris entre 700 MPa et 1000 MPa.

6. Matériau selon l'une des revendications 3 à 5, **caractérisé en ce que** la composition solide comprend majoritairement du phosphate dicalcique dihydraté $CaHPO_4, 2H_2O$.

7. Matériau selon l'une des revendications 3 à 6, **caractérisé en ce que** la composition solide est du type obtenu par mélange d'une poudre de phosphate tricalcique $\beta$ dans une solution d'acide orthophosphorique.

8. Matériau selon l'une des revendications 3 à 7, **caractérisé en ce que** la matrice est adaptée pour être entièrement résorbable dans ledit milieu, et **en ce que** la composition solide est adaptée pour présenter une cinétique de résorption dans ledit milieu plus rapide que celle de la matrice.

9. Matériau selon l'une des revendications 3 à 8, **caractérisé en ce que** la composition solide est adaptée pour être résorbable dans ledit milieu en une

**10.** Matériau selon l'une des revendications 3 à 9, **caractérisé en ce que** la matrice est une céramique macroporeuse.

**11.** Matériau selon la revendication 10, **caractérisé en ce que** la matrice présente des pores interconnectés.

**12.** Matériau selon l'une des revendications 3 à 11, **caractérisé en ce que** la matrice présente une porosité comprise entre 5 % et 85 %.

**13.** Matériau selon l'une des revendications 3 à 12, **caractérisé en ce que** la matrice est choisie pour présenter une résistance à la compression comprise entre 1 MPa et 6 MPa.

**14.** Matériau selon l'une des revendications 3 à 12, **caractérisé en ce que** la matrice est choisie pour présenter un module d'Young compris entre 10 MPa et 200 Mpa.

**15.** Matériau selon l'une des revendications 3 à 14, **caractérisé en ce que** la matrice comprend une proportion X en poids d'hydroxyapatite $Ca_{10}(PO_4)_6(OH)_2$, et une proportion Y en poids de phosphate tricalcique $Ca_3(PO_4)_2$.

**16.** Matériau selon la revendication 15, **caractérisé en ce que** :

$$X + Y = 100\ \%$$

**17.** Matériau selon la revendication 16, **caractérisé en ce que** X est de 75% et Y de 25%.

**18.** Implant **caractérisé en ce qu'**il comprend un matériau selon l'une des revendications 3 à 17.

**19.** Implant **caractérisé en ce qu'**il comprend au moins une portion en un matériau selon l'une des revendications 3 à 17, destinée à être placée au contact d'un milieu biologique humain ou animal, notamment d'un tissu osseux.

**20.** Kit de mise en oeuvre extemporanée d'un procédé selon l'une des revendications 1 et 2, **caractérisé en ce qu'**il comprend au moins un bloc d'une matrice solide poreuse biocompatible, et une dose de chacun des constituants nécessaires pour obtenir la solution liquide formant la composition solide biocompatible.

**Patentansprüche**

**1.** Verfahren zur Herstellung eines Verbundstoffs für die Implantation in das biologische Milieu eines Menschen oder Tiers, **gekennzeichnet durch** die folgenden Schritte:

- vorhergehendes Wählen oder Herstellen wenigstens eines Blocks einer festen, porösen, biokompatiblen Matrix, die mehrheitlich wenigstens ein Calciumsalz ausgewählt aus Calciumphosphaten, Calciumsulfaten und Calciumkarbonaten enthält,
- Herstellen, unabhängig von der festen Matrix, einer härtbaren flüssigen Lösung, die nach dem Härten eine feste, biokompatible Zusammensetzung bildet, die mit einem hydraulischen Bindemittel gehärtet wurde, das mehrheitlich wenigstens ein Calciumsalz ausgewählt aus Calciumphosphaten, Calciumsulfaten und Calciumkarbonaten enthält und die **durch** das Milieu, in dem das Implantat implantiert werden soll, vollkommen resorptionsfähig ist,
- Eintauchen des Matrixblocks in die flüssige Lösung vor deren Härtung für eine Dauer, die ausreicht, damit die Lösung das poröse Volumen des Matrixblocks imprägniert und bis zum Kern füllt,
- danach Trocknen- und Härtenlassen der flüssigen Lösung zu einer festen Zusammensetzung.

**2.** Verfahren nach Anspruch 1, **dadurch gekennzeichnet, dass** jeder Block des auf diese Weise erhaltenen Verbundstoffs geformt wird.

**3.** Verbundstoff für eine Implantation in ein biologisches Milieu eines Menschen oder Tiers, der mit einem Verfahren nach Anspruch 1 oder 2 erhalten werden kann, **dadurch gekennzeichnet, dass** er folgendes umfasst:

a) eine feste, poröse, biokompatible Matrix mit einer chemischen Zusammensetzung, die mehrheitlich wenigstens ein Calciumsalz ausgewählt aus Calciumphosphaten, Calciumsulfaten und Calciumkarbonaten enthält,
b) eine feste, kompakte, ungeteilte, biokompatible Zusammensetzung mit einem gehärteten hydraulischen Bindemittel, das mehrheitlich wenigstens ein Calciumsalz ausgewählt aus Calciumphosphaten, Calciumsulfaten und Calciumkarbonaten enthält, wobei diese feste Zusammensetzung das poröse Volumen der Matrix füllt und in dem genannten Milieu vollkommen resorptionsfähig ist.

durée inférieure à trois mois.

**4.** Stoff nach Anspruch 3, **dadurch gekennzeichnet, dass** die feste Zusammensetzung so gewählt wird, dass sie einen Kompressionswiderstand von mehr als 10 MPa, insbesondere zwischen 20 MPa und 50 MPa hat.

**5.** Stoff nach Anspruch 3 oder 4, **dadurch gekennzeichnet, dass** die feste Zusammensetzung so gewählt wird, dass sie ein Young sches Modul zwischen 700 MPa und 1000 MPa hat.

**6.** Stoff nach einem der Ansprüche 3 bis 5, **dadurch gekennzeichnet, dass** die feste Zusammensetzung mehrheitlich dihydriertes Dicalciumphosphat $CaHPO_4, 2H_2O$ umfasst.

**7.** Stoff nach einem der Ansprüche 3 bis 6, **dadurch gekennzeichnet, dass** die feste Zusammensetzung von einem Typ ist, der durch Mischen von $\beta$-Tricalciumphosphatpulver in einer Orthophosphorsäurelösung erhalten wird.

**8.** Stoff nach einem der Ansprüche 3 bis 7, **dadurch gekennzeichnet, dass** die Matrix in dem genannten Milieu vollkommen resorbiert werden kann, und dadurch, dass die feste Zusammensetzung eine Resorptionskinetik in dem genannten Milieu hat, die schneller ist als die der Matrix.

**9.** Stoff nach einem der Ansprüche 3 bis 8, **dadurch gekennzeichnet, dass** die feste Zusammensetzung in dem genannten Milieu über einen Zeitraum von weniger als drei Monaten resorbiert werden kann.

**10.** Stoff nach einem der Ansprüche 3 bis 9, **dadurch gekennzeichnet, dass** die Matrix eine makroporöse Keramik ist.

**11.** Stoff nach Anspruch 10, **dadurch gekennzeichnet, dass** die Matrix untereinander verbundene Poren hat.

**12.** Stoff nach einem der Ansprüche 3 bis 11, **dadurch gekennzeichnet, dass** die Matrix eine Porösität zwischen 5 und 85% hat.

**13.** Stoff nach einem der Ansprüche 3 bis 12, **dadurch gekennzeichnet, dass** die Matrix so gewählt wird, dass sie eine Kompressionsfestigkeit zwischen 1 MPa und 6 MPa hat.

**14.** Stoff nach einem der Ansprüche 3 bis 12, **dadurch gekennzeichnet, dass** die Matrix so gewählt wird, dass sie ein Young'sches Modul zwischen 10 MPa und 200 MPa hat.

**15.** Stoff nach einem der Ansprüche 3 bis 14, **dadurch gekennzeichnet, dass** die Matrix einen Gewichtsanteil X Hydroxyapatit $Ca_{10}(PO_4)_6(OH)_2$ und einen Gewichtsanteil Y Tricalciumphosphat $Ca_3(PO_4)_2$ hat.

**16.** Stoff nach Anspruch 15, **dadurch gekennzeichnet, dass**:

$$X + Y = 100\% \text{ ist.}$$

**17.** Stoff nach Anspruch 16, **dadurch gekennzeichnet, dass** X 75% und Y 25% beträgt.

**18.** Implantat, **dadurch gekennzeichnet, dass** es ein Material nach einem der Ansprüche 3 bis 17 umfasst.

**19.** Implantat, **dadurch gekennzeichnet, dass** es wenigstens einen Teil aus einem Stoff nach einem der Ansprüche 3 bis 17 umfasst, das mit einem biologischen Milieu eines Menschen oder Tieres, insbesondere einem Knochengewebe in Kontakt gebracht wird.

**20.** Garnitur zur extemporären Ausführung eines Verfahrens nach Anspruch 1 oder 2, **dadurch gekennzeichnet, dass** es wenigstens einen Block einer festen, porösen, biokompatiblen Matrix und eine Dosis jedes der Bestandteile umfasst, die notwendig sind, um die die feste biokompatible Zusammensetzung bildende flüssige Lösung zu erhalten.

**Claims**

**1.** Method for preparing a composite material to be implanted in a human or animal biological medium, **characterized in that**:

- at least one block of a biocompatible porous solid matrix is selected or previously prepared, said matrix being mainly comprised of at least one calcium salt selected from calcium phosphates, calcium sulfates and calcium carbonates,
- separate from said solid matrix, a hardenable liquid solution is prepared, said hardenable liquid solution being adapted to form, once hardened, a hardened biocompatible solid composition which is hardened by means of a hydraulic cement mainly comprised of at least one calcium salt selected from calcium phosphates, calcium sulfates and calcium carbonates, and which is adapted to be entirely resorbable by the medium wherein the implant is to be implanted,
- said matrix block is immersed in said liquid solution before the hardening thereof, during a period which allows said solution to impregnate and fill the porous volume of said matrix block to the core,
- said liquid solution is then allowed to dry and harden into a solid composition.

2. Method according to claim 1, **characterized in that** each resulting block of composite material is then shaped.

3. Composite material to be implanted in a human or animal biological medium, able to be obtained by a method according to one of claims 1 and 2, **characterized in that** it comprises:

   a) a biocompatible porous solid matrix having a chemical composition which mainly contains at least one calcium salt selected from calcium phosphates, calcium sulfates and calcium carbonates,
   b) a biocompatible, undivided, compact, solid composition of a hardened hydraulic cement which is comprised of at least one calcium salt selected from calcium phosphates, calcium sulfates and calcium carbonates, said solid composition filling the porous volume and being adapted to be entirely resorbable by the medium wherein the implant is to be implanted.

4. Material according to claim 3, **characterized in that** said solid composition is selected to have a compression strength greater than 10 MPa, and especially in the range between 20 MPa and 50 MPa.

5. Material according to one of claims 3 and 4, **characterized in that** said solid composition is advantageously selected to have a Young modulus in the range between 700 MPa and 1000 MPa.

6. Material according to one of claims 3 to 5, **characterized in that** said solid composition is mainly comprised of dihydrated dicalcium phosphate $CaHPO_4.2H_2O$.

7. Material according to one of claims 3 to 6, **characterized in that** said solid composition is of the type obtained by mixing a powdered $\beta$-tricalcium phosphate in an orthophosphoric acid solution

8. Material according to one of claims 3 to 7, **characterized in that** said matrix is adapted to be entirely resorbable in said medium, and said solid composition is adapted to have a resorption kinetics in said medium quicker than said matrix.

9. Material according to one of claims 3 to 8, **characterized in that** said solid composition is adapted to be resorbable in said medium within a period shorter than three months.

10. Material according to one of claims 3 to 9, **characterized in that** said matrix is a macroporous ceramic.

11. Material according to claim 10, **characterized in that** said matrix has interconnected pores.

12. Material according to one of claims 3 to 11, **characterized in that** said matrix has a porosity which is in the range between 5 % and 85 %.

13. Material according to one of claims 3 to 12, **characterized in that** said matrix is selected to have a compression strength in the range between 1 MPa and 6 MPa.

14. Material according to one of claims 3 to 12, **characterized in that** said matrix is selected to have a Young modulus in the range between 10 MPa and 200 MPa.

15. Material according to one of claims 3 to 14, **characterized in that** said matrix contains a weight proportion X of hydroxyapatite $Ca_{10}(PO_4)_6(OH)_2$, and a weight proportion Y of tricalcium phosphate $Ca_3(PO_4)_2$.

16. Material according to claim 15, **characterized in that** X + Y = 100 %.

17. Material according to claim 16, **characterized in that** X is of 75 % and Y of 25 %.

18. Implant **characterized in that** it comprises a material according to one of claims 3 to 17.

19. Implant **characterized in that** it comprises at least a portion in a material according to one of the claims 3 to 17, which is to be placed in contact with a human or animal biological medium, especially a bone tissue.

20. Kit for carrying out extemporaneoulsy a method according to one of claims 1 and 2, **characterized in that** it comprises at least one block of a biocompatible porous solid matrix and one dose of each of the constituents necessary to obtain said liquid solution which forms said biocompatible solid composition.